# EUROPÄISCHE PATENTSCHRIFT

(11) **EP 4 213 899 B1**
(45) Veröffentlichungstag und Bekanntmachung des Hinweises auf die Patenterteilung: **04.11.2026**
(21) Anmeldenummer: 21777464.5
(22) Anmeldetag: 10.09.2021
(51) Int. Cl.: A61L 24/00, A61L 24/10

(54) **GEWEBEKLEBER ZUR ANWENDUNG IN EINEM BEHANDLUNGSVERFAHREN, BEI WELCHEM EINEM MENSCHLICHEN ODER TIERISCHEN PATIENTEN EIN OPHTHALMOLOGISCHES IMPLANTAT IMPLANTIERT WIRD, UND OPHTHALMOLOGISCHES IMPLANTATIONSSYSTEM**
TISSUE ADHESIVE FOR USE IN A TREATMENT METHOD IN WHICH AN OPHTHALMOLOGICAL IMPLANT IS IMPLANTED IN A HUMAN OR ANIMAL PATIENT, AND OPHTHALMOLOGICAL IMPLANTATION SYSTEM
ADHÉSIF POUR TISSUS DESTINÉ À ÊTRE UTILISÉ DANS UNE MÉTHODE DE TRAITEMENT DANS LAQUELLE UN IMPLANT OPHTALMOLOGIQUE EST IMPLANTÉ CHEZ UN PATIENT HUMAIN OU ANIMAL, ET SYSTÈME D'IMPLANTATION OPHTALMOLOGIQUE

(30) Priorität: 18.09.2020 DE 102020124372
(43) Veröffentlichungstag der Anmeldung: 26.07.2023
(73) Patentinhaber: Carl Zeiss Meditec AG, 07745 Jena (DE)
(72) Erfinder: WOLFSTEIN, Andre, 13507 Berlin (DE)
(74) Vertreter: Hofstetter, Schurack & Partner
(86) Internationale Anmeldenummer: PCT/EP2021/074960
(87) Internationale Veröffentlichungsnummer: WO 2022/058247

(56) Entgegenhaltungen:
- WO-A1-2013/040559
- WO-A2-2006/067638
- US-A1- 2011 287 067
- US-A1- 2019 070 338
- US-B1- 6 702 853
- YI HONG ET AL: "A strongly adhesive hemostatic hydrogel for the repair of arterial and heart bleeds", NATURE COMMUNICATIONS, vol. 10, no. 1, 14 May 2019 (2019-05-14), XP055707391, DOI: 10.1038/s41467-019-10004-7

## Beschreibung

### Technisches Gebiet

Die Erfindung betrifft einen Gewebekleber zur Anwendung in einem Behandlungsverfahren, bei welchem einem menschlichen oder tierischen Patienten ein ophthalmologisches Implantat implantiert wird, sowie ein ophthalmologisches Implantationssystem für ein derartiges Behandlungsverfahren.

### Stand der Technik

Bei der Verwendung von ophthalmologischen Implantaten, beispielsweise von Intraokularlinsen (IOLs) oder künstlichen Kapselsäcken, können aufgrund von Wechselwirkungen zwischen dem Implantat und angrenzendem biologischem Gewebe verschiedene Komplikationen auftreten. Beispielsweise tritt nach Katarakt-Operationen in manchen Fällen eine sogenannte posteriore Kapselopazifikation (posterior capsule opacification, PCO, Cataracta secundaria) auf. Bei PCO handelt es sich um eine postoperative Trübung der Linsenkapsel nach der chirurgischen Extraktion einer natürlichen Linse. Die verbleibenden Linsenepithelzellen (E-Zellen) in der äquatorialen Region des Kapselsacks sind mitotisch aktiv und können zu Fibroblasten transformieren. Diese lösen dann eine Art Wundheilung aus, wobei kollagenhaltiges Bindegewebe gebildet wird. Da manche Fibroplasten-Subtypen nicht nur an die Innenseite des Kapselsacks migrieren, sondern sich auch zusammenziehen können, kommt es zur Faltenbildung im Kapselsack. Die Trübung der Kapsel ist somit die Folge eines Wundheilungsprozesses und einer damit verbundenen Narbenbildung. Da die dadurch verursachte Linsentrübung andere Ursachen als die ursprüngliche Katarakt-Erkrankung besitzt, spricht man von einem "Nachstar" oder einer "sekundären Katarakt". Ein klinisch signifikanter Nachstar kann bei den Betroffenen zu einer Minderung der Sehschärfe, der Farbwahrnehmung und des Kontrastsehens sowie zu einer erhöhten Blendung führen.

Beim sekundären Katarakt handelt es sich um eine häufige Komplikation nach extrakapsulären Kataraktoperation (ECCE) und der anschließenden Implantation einer Intraokularlinse (IOL) in den Kapselsack. Ohne die Implantation einer IOL in den leeren Kapselsack ist das Risiko eines Nachstars sogar noch erheblich erhöht, da in diesem Fall eine ungehinderte Zellmigration zur hinteren Oberfläche des Kapselsackes möglich ist. Auch die Implantation eines künstlichen Kapselsacks birgt ein vergleichsweise hohes Risko eines Nachstars oder anderer Komplikationen, die durch unkontrolliertes Zellwachstum verursacht werden.

Die Inzidenz eines sekundären Katarakts erhöht sich nach einem operativen Eingriff mit der Zeit. Eine Meta-Analyse der Fälle von Cataracta secundaria für alle bestehenden Arten von IOLs zeigte postoperativ eine ungefähre durchschnittliche Zunahme von 12% nach einem Jahr und eine ungefähre durchschnittliche Zunahme von 30% nach fünf Jahren. Ein entscheidender Faktor scheint hierbei auch das Alter des betreffenden Patienten zu sein, so dass damit gerechnet werden muss, dass fast alle behandelten Kinder und Jugendlichen nach einer gewissen Zeit unter postoperativem sekundären Katarakt leiden könnten.

Die US 2011/287067 A1 offenbart die Synthese von verstärkten Klebstoffkomplexen und deren Verwendung. Die verstärkten Klebstoffkomplexe bestehen aus mindestens einem Polykation, mindestens einem Polyanion und einer Verstärkungskomponente. Die beschriebenen Klebstoffkomplexe können anschließend gehärtet werden, um starke, kohäsive Klebstoffe herzustellen.

Die US 2019/070338 A1 offenbart neurounterstützende Materialien, die eine starke Gewebeadhäsion aufweisen und durch Vernetzung zweier Polymere, Gelatine-Methacryloyl (GelMA) und Methacryloyl-substituiertes Tropoelastin (MeTro), synthetisiert wurden. Die entwickelten Materialien weisen durch Variation des GelMA/MeTro-Verhältnisses einstellbare mechanische Eigenschaften auf.

Yi Hong et al.:"A strongly adhesive hemostatic hydrogel for the repair of arterial and heart bleeds" (Nature Communications, Bd. 10, Nr. 1, 14. 2019-05-14, XP055707391) offenbart einen photoreaktiven Klebstoff, der die Zusammensetzung der extrazellulären Matrix (ECM) nachahmt. Dieses auf Biomakromolekülen basierende Matrix-Hydrogel kann schnell gelieren und fixiert werden, um blutende Arterien und Herzwände nach UV-Licht-Bestrahlung zu verkleben und abzudichten.

Die US 6 702 853 B1 offenbart ein System und ein Verfahren zum Entfernen von Kataraktzellen in einer Linsenkapsel eines Auges und zum Einsetzen einer Intraokularlinse in die Linsenkapsel, wobei auf mindestens eine ihrer Oberflächen ein Klebstoff aufgebracht ist.

Vorzugsweise wird der Klebstoff auf die Oberfläche der Intraokularlinse aufgebracht, die der Hornhaut des Auges zugewandt ist.

Die WO 2006/067638 A2 offenbart amphiphile Blockcopolymere, die mindestens einen Block mit hydrophilen Einheiten und mindestens einen Block mit hydrophoben Einheiten umfassen, wobei mindestens ein hydrophober Block Siloxaneinheiten enthält. Die Blockcopolymere können insbesondere als Gewebekleber oder als Beschichtung für eine Intraokularlinse (IOL) nützlich sein. Als IOL-Beschichtung können die Blockcopolymere beispielsweise verwendet werden, um die Gewebeadhäsion zur Verhinderung einer hinteren Kapseltrübung zu fördern.

Es ist bisher nicht möglich, im Rahmen von Augenoperationen alle Epithelzellen quantitativ zu entfernen, ohne toxische Arzneimittel zu verwenden, die die Endothelzellschicht der Hornhaut beschädigen oder anderes Augengewebe schädigen können. Die durch Fibrose und PCO hervorgerufenen Sehprobleme werden teilweise durch eine sogenannte fokale Nd:YAG-Kapsulotomie behandelt. Trotzdem können insbesondere fibrotische Striae weiterhin die Funktion von Implantaten wie beispielsweise einer akkommodationsfähigen IOL beeinträchtigen.

Bis zu diesem Zeitpunkt konnte daher keine signifikante und langfristige prophylaktische Wirkung gegen Cataracta secundaria und andere medizinischen Komplikationen, die im Zusammenhang mit der Implantation von ophthalmologischen Implantaten auftreten, erzielt werden.

### Darstellung der Erfindung

Aufgabe der vorliegenden Erfindung ist es, das Risiko von PCO und Fibrose für Behandlungsverfahren, bei welchem einem menschlichen oder tierischen Patienten ein ophthalmologisches Implantat implantiert wird, zu verringern. Eine weitere Aufgabe der Erfindung ist es, ein ophthalmologisches Implantationssystem für ein derartiges Behandlungsverfahren zu schaffen, welches das Risiko von PCO und Fibrose verringern kann.

Die Aufgaben werden erfindungsgemäß durch einen Gewebekleber gemäß Patentanspruch 1 zur Anwendung in einem Behandlungsverfahren, bei welchem einem menschlichen oder tierischen Patienten ein ophthalmologisches Implantat implantiert wird, sowie durch ein ophthalmologisches Implantationssystem gemäß Patentanspruch 6 gelöst. Vorteilhafte Ausgestaltungen mit zweckmäßigen Ausbildungen der Erfindung sind in den jeweiligen Unteransprüchen angegeben, wobei vorteilhafte Ausgestaltungen jedes Erfindungsaspekts als vorteilhafte Ausgestaltungen der jeweils anderen Erfindungsaspekte anzusehen sind.

Ein erster Aspekt der Erfindung betrifft einen Gewebekleber gemäß Anspruch 1 zur Anwendung in einem Behandlungsverfahren, bei welchem einem menschlichen oder tierischen Patienten ein ophthalmologisches Implantat implantiert und das ophthalmologische Implantat mittels des Gewebeklebers zur Verringerung des Risikos von posteriorer Kapselopazifikation (PCO) und Fibrose zumindest teilweise stoffschlüssig mit Augengewebe des Patienten verbunden wird, wobei der Gewebekleber im nicht-ausgehärteten Zustand als interpenetrierendes Netzwerk und/oder als semi-interpenetrierendes Netzwerk vorliegt. Mit anderen Worten wird erfindungsgemäß ein Gewebekleber bereitgestellt, welcher im Rahmen eines Behandlungsverfahrens, bei welchem beispielsweise eine Augenlinse eines Patienten durch ein ophthalmologisches Implantat ersetzt wird, dazu verwendet werden kann, das ophthalmologische Implantat teilweise oder vollständig stoffschlüssig mit einem Kapselsack des Patienten zu verbinden. Entsprechend kann der Gewebekleber zum stoffschlüssigen Verbinden eines künstlichen Kapselsacks mit Augengewebe des Patienten sowie für weitere Augenimplantationsarten verwendet werden. Unter einem Gewebekleber wird im Rahmen der vorliegenden Erfindung ein pharmakologisch tolerierbarer Klebstoff verstanden, welcher dazu ausgebildet ist, in vivo eine stoffschlüssige Verbindung zwischen dem biologischen Augengewebe und dem an das betreffende Augengewebe anliegenden ophthalmologischen Implantat auszubilden. Unter dem Begriff "stoffschlüssig" werden im Rahmen der vorliegenden Offenbarung Verbindungen verstanden, bei denen die Verbindungspartner durch atomare oder molekulare Bindungskräfte zusammengehalten werden, wobei die Bindungskräfte starke Bindungen und/oder schwache Bindungen (Van-der-Waals-Wechselwirkungen) beinhalten können. Vorzugsweise umfassen die Bindungskräfte starke Bindungen, insbesondere kovalente Bindungen. Ein Gewebekleber im Rahmen der vorliegenden Offenbarung ist damit kein reiner Haftvermittler wie beispielsweise Fibronectin, Vitronectin, Laminin oder Glycoproteine, sondern erzeugt unter chemischer Aushärtung eine stoffschlüssige Verbindung zwischen dem betreffenden Augengewebe und dem Implantat. Gewebekleber sind an sich für abweichende chirurgische Verfahren für den Wundverschluss ohne chirurgisches Nahtmaterial bekannt, aber bislang nicht für die erfindungsgemäße medizinische Indikation im Rahmen einer gattungsgemäßen Augenoperation beschrieben. Mit Hilfe des erfindungsgemäßen Gewebeklebers kann das ophthalmologische Implantat im Auge immobilisiert und fest gebunden werden, ohne das Augengewebe zu schädigen. Damit können bei gattungsgemäßen Behandlungsverfahren häufig auftretende Probleme wie Dezentrierung, Kippung, Rotation oder Ablösung des Implantats zuverlässig verhindert werden. Darüber hinaus kann bislang eine solche kapselsackbasierte Implantation mit einer hinteren Kapseltrübung (PCO) und Fibrose assoziiert sein, die insbesondere durch verbleibende Linsenepithelzellen verursacht werden kann. Durch die Möglichkeit der stoffschlüssigen Anbindung des Implantats an das Augengewebe mit Hilfe des Gewebeklebers kann eine Unterwanderung des Implantats mit Linsenepithelzellen und dergleichen hingegen zuverlässig verhindert werden. Eventuell bei der Operation im Auge verbliebene Linsenepithelzellen und sonstige Zelltypen können damit keine PCO und Fibrose oder ähnliche Komplikationen mehr verursachen, die die Sicht und Funktionalität eines Implantats, beispielsweise einer Intraokularlinse (IOL), insbesondere einer akkommodierenden IOL, oder eines künstlichen Kapselsacks beeinträchtigen können. Indem der Gewebekleber im nicht-ausgehärteten Zustand als interpenetrierendes Netzwerk und/oder als semi-interpenetrierendes Netzwerk vorliegt, können vorteilhaft Netzwerkkomponenten verwendet werden, die chemisch identisch oder verschieden sein können. Zudem können auf diese Art zwei an sich nicht mischbare Polymertypen verwendet und miteinander verbunden werden. Weiterhin können Monomere, die nach völlig unterschiedlichen, nicht interferierenden Mechanismen polymerisieren, einer simultanen interpenetrierenden Vernetzung unterzogen werden. Hierdurch kann die Art und Initiierung der Aushärtung optimal an den jeweiligen Anwendungszweck angepasst werden. Generell sind "ein/eine" im Rahmen dieser Offenbarung als unbestimmte Artikel zu lesen, also ohne ausdrücklich gegenteilige Angabe immer auch als "mindestens ein/mindestens eine". Umgekehrt können "ein/eine" auch als "nur ein/nur eine" verstanden werden. Der Begriff "umfassen" ist im Rahmen der vorliegenden Offenbarung generell so auszulegen, dass die entsprechenden Merkmale enthalten sind, das Vorhandensein anderer Merkmale aber nicht ausgeschlossen ist. Umgekehrt kann der Begriff "umfassen" im Rahmen der vorliegenden Offenbarung aber auch im Sinne von "bestehend aus" bzw. von "bestehend im Wesentlichen aus" ausgelegt werden, dass heißt dass neben den im Anschluss an diese Formulierung genannten Merkmalen keine weiteren Merkmale ("bestehen aus") vorhanden sein können oder dass bestimmte weitere Merkmale vorhanden sein können, nämlich solche, die die wesentlichen Merkmale des Erfindungsgegenstands nicht signifikant verändern ("bestehend im Wesentliche aus").

In der vorliegenden Erfindung ist vorgesehen, dass der Gewebekleber als Zusammensetzung mit einem Wirkstoffabgabesystem bereitgestellt ist, welches dazu ausgebildet ist, im implantierten Zustand des Gewebeklebers wenigstens einen pharmakologischen Wirkstoff, vorzugsweise kontrolliert, abzugeben und optional dass der Gewebekleber als Zusammensetzung mit einem am Gewebekleber immobilisierten pharmakologischen Wirkstoff bereitgestellt ist. Hierdurch kann der Gewebekleber nach der Implantation vorteilhaft zur lokalen Abgabe von einem oder mehreren pharmakologisch wirksamen Substanzen bzw. zur langanhalten Positionierung des Wirkstoffs an einem gewünschten Ort verwendet werden. Hierdurch können ein oder mehrere Wirkstoffe - vorzugsweise kontrolliert - gezielt in den Bereich abgegeben werden, in dem sie benötigt werden. Eine systemische bzw. unspezifische Gabe des Wirkstoffs und damit verbundene Nebenwirkungen können vorteilhaft vermieden werden. Beispielsweise kann der Wirkstoff Thapsigargin bei freier Abgabe in das Kammerwasser der Vorderkammer die Epithelzellschicht der Hornhaut schädigen. Demgegenüber sorgt die vorzugsweise kontrollierte Abgabe in das direkt angrenzende Gewebe für die Beschränkung der pharmakologischen Wirkung auf den Ort, wo diese benötigt wird. Weiterhin kann die Menge des Wirkstoffs auf eine therapeutisch notwendige Menge reduziert werden, wodurch trotz einer verbesserten und langanhaltender Wirksamkeit eine Senkung der Komplikationsrisiken sowie erhebliche Kosteneinsparungen realisierbar sind.

Weitere Vorteile ergeben sich dadurch, dass der wenigstens eine Wirkstoff zum Fördern und/oder Hemmen wenigstens eines Aspekts aus einer Gruppe, die Proliferation, Migration und Differenzierung von im menschlichen oder tierischen Auge vorkommenden Zellen aufweist, ausgebildet ist. Mit anderen Worten ist der Wirkstoff dazu ausgebildet, das Entstehen von PCO bzw. Fibrose zu kontrollieren bzw. zu steuern, indem die Proliferation, Migration und/oder Differenzierung von Zellen wie beispielsweise Linsenepithelzellen im implantierten Zustand gezielt gefördert und/oder gehemmt wird. Die Hemmung der Umwandlung, des Zellwachstums und/oder der Zellteilung von derartigen Zellen verhindert ebenfalls vorteilhaft eine Unter- bzw. Hinterwanderung oder Überwucherung des mittels des Gewebeklebers im Augengewebe "festgeklebten" Implantats, wodurch das Entstehen von PCO bzw. Fibrose sowie verwandter Komplikationen besonders effektiv unterdrückt und verhindert werden kann. Alternativ oder zusätzlich kann vorgesehen sein, dass die Proliferation, Migration und/oder Differenzierung von Zellen wie beispielsweise Linsenepithelzellen gezielt gefördert werden. Dies bewirkt ein verstärktes Gewebewachstum durch eine aktive Zellkultivierung und fördert dadurch eine natürliche Wundheilung. Die Proliferation und Migration der im menschlichen oder tierischen Auge vorkommenden Zellen können damit kontrolliert werden, so dass diese Zellen die vorstehend genannten Probleme, die beispielsweise zur PCO führen, nicht länger verursachen können. Darüber hinaus ist die Induzierung und gegebenenfalls Steuerung einer schnellen Fibrose von Vorteil, da sie die Stabilität des Implantats erhöht, die endgültige Positionierung des Implantats im Augengewebe schnell sicherstellt und die Heilungsdauer beispielsweise nach der Kataraktoperation oder nach dem Einsetzen eines künstlichen Kapselsacks verkürzt. Die Zellen können dabei grundsätzlich auch in verschiedenen Zellformen, beispielsweise als Fibroplasten, und/oder als Zellansammlungen, beispielsweise als Wedl-Zellen auftreten.

In der vorliegenden Erfindung ist vorgesehen, dass der wenigstens eine Wirkstoff ausgewählt ist aus einer Gruppe, die 5-Fluoruracil, Thapsigargin, Paclitaxel, Wachstumsfaktoren, insbesondere TGFβ, und Angiogenesehemmer sowie Derivate, , insbesondere (Meth)Acrylat-modifizierte Derivate, Isomere und beliebige Mischungen hiervon umfasst. 5-Fluoruracil ist ein Zytostatikum und gehört als Pyrimidinanalogon zur Gruppe der Antimetabolite. Es besitzt eine strukturelle Ähnlichkeit mit der Pyrimidinbase Uracil und wird an ihrer Stelle in die RNA eingebaut. Darüber hinaus hemmt 5-Fluorouracil ein Schlüsselenzym der Pyrimidinbiosynthese, die Thymidilat-Synthase. Weiterhin kann als Wirkstoff das Zytotoxin modifiziertes oder unmodifiziertes Thapsigargin vorgesehen sein. Thapsigargin ist ein Inhibitor des Calcium-ATPase-Inhibitors des endoplasmatischen Retikulums, das Zellwachstum im Kapselsack bei niedrigen Konzentrationen (100 nM) stark reduziert und bei höheren Konzentrationen (10-100 µM) den Zelltod induziert. Vorzugsweise ist das Thapsigargin (Meth)Acrylat-modifiziert, um eine sterisch ungehinderte kovalente Bindung an Doppelbindungen sicherzustellen. Thapsigargin kann beispielsweise durch Reaktion mit (Meth)Acrylanhydrid entsprechend derivatisiert werden. Unter dem Begriff "(Meth)Acrylat" sind im Rahmen der Offenbarung sowohl Acrylate als auch Methacrylate sowie Mischungen hieraus zu verstehen. Paclitaxel ist ein Spindelgift und hemmt durch eine Bindung an β-Tubulin den Abbau von Spindelfasern, welche aus Mikrotubuli aufgebaut sind. Dadurch wird die mitotische Zellteilung in der G2- und M-Phase blockiert, so dass eine Zellproliferation unterbleibt. Die genannten Verbindungen und ihre Isomere und/oder Derivate können somit, einzeln und in beliebiger Kombination, insbesondere zur Hemmung der Proliferation, Migration und Differenzierung von im menschlichen oder tierischen Auge vorkommenden Zellen verwendet werden. Alternativ oder zusätzlich können ein oder mehrere Wachstumsfaktoren vorgesehen sein, um eine Wundheilungsreaktion zu fördern. Der Wachstumsfaktor kann beispielsweise TGF-β (TGF-β1, TGF-β2, TGF-β3) sein oder umfassen. Weiterhin können als Wirkstoff(e) ein oder mehrere Angiogenesehemmer vorgesehen sein. Der Angiogenesehemmer kann ausgewählt sein aus einer Gruppe, die VEGF-Inhibitoren, VEGFR-Inhibitoren, Antikörper, Fab-Fragmente, variable Einzelkettenfragmente (scFv), multivalente Antikörperfragmente (scFv Multimere), Peptid-Aptamere und Peptide umfasst. VEGF-Inhibitoren sind eine Gruppe strukturell unterschiedlicher Arzneistoffe, die an den Wachstumsfaktor VEGF binden und dadurch die Angiogenese hemmen. VEGFR-Inhibitoren sind demgegenüber Arzneistoffe, die an den VEGF-Rezeptor (VEGFR) binden. Dabei kann es sich um Small molecules aus der Gruppe der Tyrosinkinaseinhibitoren (TKI) oder um Antikörper, insbesondere um monoklonale Antikörper handeln. In beiden Fällen kommt es zur Unterbrechung der intrazellulären Signalkaskade, wodurch eine Hemmung der Angiogenese bewirkt wird. Alternativ oder zusätzlich können Fab-Fragmente, variable Einzelkettenfragmente (scFv), multivalente Antikörperfragmente (scFv Multimere), Peptid-Aptamere und Peptide vorgesehen sein, die die Angiogenese unterbinden oder zumindest signifikant verlangsamen. Hierdurch kann eine optimal auf das jeweilige Krankheitsbild angepasste Selektionierung durchgeführt werden. Antikörperfragmente bieten den Vorteil einer hohen Bindungsaffinität bzw. -avidität und Spezifität für ein breites Spektrum an biologischen Zielstrukturen und Haptenen. Einzelkettenfragmente können zudem als Diabodies (60 kDa), Triabodies (90 kDa), Tetrabodies (120 kDa) etc. vernetzt bzw. exprimiert werden, wobei unterschiedliche Linkerlängen zwischen V-Domänen möglich sind. Ein besonderer Vorteil ist zudem, dass Moleküle von 60-120 kDa die Penetration von Zellen erhöhen und schnellere Clearance-Raten als korrespondierende Igs (150 kDa) besitzen. Es kann weiterhin vorgesehen sein, dass der Antikörper ein Diabody, Triabody, Tetrabody, Pentabody, Hexabody, Heptabody oder Octabody ist. Mit anderen Worten ist es vorgesehen, dass der Antikörper mono-, bi-, tri-, quad-, pent-, hex-, hept-, oct-, enn- oder multispezifisch ausgebildet ist. Dies erlaubt die Quervernetzung von zwei, drei, vier, fünf, sechs, sieben oder acht Zielstrukturen bzw. Zielproteinen, wobei scFv Multimere besonders präzise und individuell an die gegebenenfalls patientenspezifische räumliche Anordnung der Zielepitope angepasst werden können, um die Angiogenese zu unterbinden. Die erhöhte Bindungswertigkeit von scFv-Multimeren führt zu einer besonders hohen Avidität. Der wenigstens eine Angiogenesehemmer kann insbesondere ausgewählt sein aus einer Gruppe, die Bevacizumab, Brolucizumab, Ranibizumab, Ramucirumab, Aflibercept, Pegaptanib, Thalidomid, Axitinib, Lenvatinib, Lucitanib, Motesanib, Pazopanib, Regorafenib, Sorafenib, Sunitinib, Tivozanib, Vatalanib und Biosimilars davon umfasst. Hierdurch können insbesondere Tyrosinkinasen gehemmt werden, wobei manche der genannten Verbindungen als Multikinase-Inhibitoren mehrerer Proteinkinasen verschiedener Klassen hemmen können und dadurch eine verbesserte Wirksamkeit aufweisen können.

Weitere Vorteile ergeben sich dadurch, dass der Wirkstoff kovalent, insbesondere über eine (Meth)Acrylat-Gruppe, an den Gewebekleber gebunden ist und/oder dass der Wirkstoff und der Gewebekleber als interpenetrierendes Netzwerk vorliegen. Durch eine kovalente Anbindung, gegebenenfalls unter Verwendung eines (Meth)Acrylats bzw. eines Spacers, kann eine besonders einfache und flexible Immobilisierung des Wirkstoffs an den Gewebekleber erfolgen, ohne dass es zu sterischer Hinderung kommt. Darüber hinaus kann das (Meth)Acrylat bzw. der Spacer in Abhängigkeit der jeweiligen Ausgestaltungsform die notwendigen funktionellen Gruppen bereitstellen, um die kovalente Anbindung des Wirkstoffs an den Gewebekleber zu ermöglichen. Dies kann in manchen Ausführungsformen beispielsweise auch durch Pfropfpolymerisation erreicht werden. Generell können aber auch andere Immobilisierungstechniken wie beispielweise interpenetrierende bzw. semi-/pseudointerpenetrierende Netzwerke und dergleichen vorgesehen sein.

In einer weiteren vorteilhaften Ausgestaltung der Erfindung liegt der Gewebekleber im nicht-ausgehärteten Zustand als Hydrogel vor. Alternativ oder zusätzlich ist der Gewebekleber durch wenigstens einen Mechanismus aus der Gruppe anaerob aushärtend, durch UV-Licht aushärtend, anionisch aushärtend, mit Aktivator aushärtend, durch Feuchtigkeit aushärtend und warmhärtend auszuhärten. Hierdurch kann die Art und Initiierung der Aushärtung optimal an den jeweiligen Anwendungszweck angepasst werden. Bevorzugt ist eine UV-initiierte Aushärtung.

Weitere Vorteile ergeben sich, indem der Gewebekleber ein MeTro (methacryliertes rekombinantes Tropoelastin) Präpolymer und/oder ein GelMA (methacrylierte Gelatine)/HA-NB (N-(2-aminoethyl)-4-(4-(hydroxymethyl)-2-methoxy-5-nitrosophenoxy)butanamid)-enthaltendes Polymer umfasst. Hierdurch wird ein hochelastischer, stark haftender und biokompatibler Gewebekleber realisiert, der gut an dem weichem Gewebe des Kapselsacks haftet. Die Herstellung eines geeigneten MeTro-Präpolymers ist allgemein beispielsweise aus Annabi et al. ("Engineering a highly elastic human protein-based sealant for surgical applications", Sci. Transl. Med. 9, eaai7466 (2017)) bekannt. MeTro-Präpolymere können unter Verwendung von rekombinantem humanem Tropoelastin und Methacrylsäureanhydrid synthetisiert werden. Beispielsweise können MeTro-Prepolymere mit einem Methacryloyl-Substitutionsgrad von 54 % (niedrig), 76 % (mittel) und 82 % (hoch) unter Verwendung von 8, 15 bzw. 20 % (v/v) Methacrylsäureanhydrid synthetisiert werden. Grundsätzlich sind aber auch abweichende Gewichts- bzw. Volumenanteile möglich. Die gebildeten MeTro-Hydrogele können dann durch Photovernetzung mit UV-Licht (6,9 mW/cm²; 360 bis 480 nm) bei verschiedenen Belichtungszeiten von 30 bis 180 s ausgehärtet werden. Als Photoinitiator kann beispielsweise [2-Hydroxy-1-(4-(Hydroxyethoxy)phenyl)-2-methyl-1-propanon (Irgacure 2959); 0,5%, w/v] verwendet werden. Alternativ oder zusätzlich kann der Gewebekleber ein GelMA (methacrylierte Gelatine)/NB (N-(2-aminoethyl)-4-(4-(hydroxymethyl)-2-methoxy-5-nitrosophenoxy)butanamid)-enthaltendes Polymer umfassen bzw. aus einem solchen bestehen. Hierbei handelt es sich um ein photoreaktives Polymer, das die Zusammensetzung der extrazellulären Matrix (ECM) nachahmt. Dieses ebenfalls auf Biomakromolekülen basierende Matrix-Hydrogel kann nach UV-Lichtbestrahlung schnell ausgehärtet werden, um das Implantat mit dem Kapselsack zu verkleben. Dieses Polymer kann zusätzlich mittels bis zu 0,1 % oder mehr eines Polymerisationsinitiators, beispielsweise Lithium-Phenyl-2,4,6-trimethylbenzoylphosphinat (LAP), an ein hydrophiles Polymer gebunden sein, das vorzugsweise aus einer Gruppe ausgewählt ist, die Alginsäure, Carboxymethylcellulose, Chitosan, Dextran, Dextransulfat, Pentosanpolysulfat, Carrageenan, Pektin, Pektinderivate, Cellulose, Cellulosederivate, Glucosaminoglykane, insbesondere Hyaluronsäure, Dermatansulfat, Chondroitinsulfat, Dermatansulfat, Keratansulfat, Heparansulfat, Heparin, Heparansulfat, Hyaluronan, Agarose, Stärke, Methylcellulose, Polymannuronsäure, Polyguluronsäure, Polyglucuronsäure, Amylose, Amylopektin, Callose, Polygalactomannan, Xanthan, Poly(ethylenoxid), Poly(ethylenglykol), Kollagen, Gelatine, Fibrin, Fibrinogen, Fibronektin, Vitronectin, Poly(ethylenoxid), Poly(acrylsäure), Poly(methacrylsäure), Poly(acrylamid), Polyvinylpyrrolidon, Poly(aminosäuren); Poly(amine), Poly(imine), eine Mischung hieraus und/oder Copolymerisate hiervon und/oder pharmakologisch akzeptable Salze hiervon umfasst. Beispielsweise kann das hydrophile Polymer Hyaluronsäure (HA) sein. Ein geeigneter Gewebekleber und seine Herstellung sind beispielsweise aus Hong, Y., Zhou, F., Hua, Y. et al. (A strongly adhesive hemostatic hydrogel for the repair of arterial and heart bleeds. Nat Commun 10, 2060 (2019)) bekannt (vgl. Seite 7-9, Methods). Beispielsweise kann der Gewebekleber 1 %-10 %, insbesondere 5 % methacrylisierter Gelatine (GelMA) und 0,5 %-3 %, insbesondere 1,25 % N-(2-Aminoethyl)-4-(4-(hydroxymethyl)-2-methoxy-5-nitrosophenoxy)butanamid (NB) umfassen, wobei das NB mittels LAP an HA gebunden ist (HA-NB). Generell sind Prozentangaben im Rahmen der vorliegenden Offenbarung als Massenprozente aufzufassen, sofern nichts anderes angegeben ist.

Ein zweiter Aspekt der Erfindung betrifft ein Implantationssystem gemäß Anspruch 6, welches das Risiko von PCO und Fibrose verringern kann, indem es ein ophthalmologisches Implantat zur Implantation in einem menschlichen oder tierischen Auge und einen Gewebekleber, mittels welchem das ophthalmologische Implantat zur Verringerung des Risikos von posteriorer Kapselopazifikation (PCO) und Fibrose zumindest teilweise stoffschlüssig mit Augengewebe des Patienten verbindbar ist, umfasst. Mit Hilfe des erfindungsgemäßen Implantationssystem kann das ophthalmologische Implantat im Augengewebe, beispielsweise im Kapselsack, immobilisiert und fest gebunden werden, ohne das Gewebe zu schädigen. Damit können im Rahmen entsprechender Behandlungsverfahren häufig auftretende Probleme wie Dezentrierung, Kippung, Rotation oder Ablösung des Implantats zuverlässig verhindert werden. Darüber hinaus kann im Fall einer Kataraktoperation eine solche kapselsackbasierte Implantation bislang mit einer hinteren Kapseltrübung (PCO) und Fibrose assoziiert sein, die insbesondere durch verbleibende Linsenepithelzellen in der Äquatorialregion des Kapselsacks verursacht werden kann. Durch die Möglichkeit der stoffschlüssigen Anbindung des Implantats an den Kapselsack mit Hilfe des Gewebeklebers kann eine Unterwanderung des Implantats mit Linsenepithelzellen und dergleichen hingegen zuverlässig verhindert werden. Eventuell verbliebene Linsenepithelzellen des für die Implantation vorbereiteten Kapselsacks können damit keine PCO und Fibrose mehr verursachen, die die Sicht und Funktionalität eines Implantats beeinträchtigen können. In bestimmten Ausführungsformen weist das Implantat einen Grundkörper mit wenigstens einem haptischen Teil und wenigstens einem optischen Teil auf, wobei der Gewebekleber nur auf dem haptischen, nur auf dem optischen oder auf beiden Teilen angeordnet werden kann. Der Gewebekleber kann grundsätzlich getrennt vom ophthalmologischen Implantat vorliegen, beispielsweise in einer separaten Verpackung, oder bereits zumindest auf einem Teil des Implantats aufgebracht sein. Weitere Merkmale und deren Vorteile sind den Beschreibungen des vorstehenden Erfindungsaspekts zu entnehmen.

In der vorliegenden Erfindung ist vorgesehen, dass der Gewebekleber gemäß dem ersten Erfindungsaspekt ausgebildet ist und optional dass das ophthalmologische Implantat eine Intraokularlinse (IOL), insbesondere eine akkommodierende IOL, oder ein künstlicher Kapselsack ist. Ohne die Implantation einer IOL in den leeren Kapselsack ist das Risiko eines Nachstars sogar erhöht, da in diesem Fall eine ungehinderte Zellmigration zur hinteren bzw. posterioren Oberfläche des Kapselsackes möglich ist.

Weitere Vorteile ergeben sich dadurch, dass das ophthalmologische Implantat an seiner Außenseite freie Aminogruppen umfasst, über welche das ophthalmologische Implantat vermittels des Gewebeklebers stoffschlüssig mit dem Kapselsack verbindbar ist. Mit anderen Worten ist es vorgesehen, dass der Gewebekleber und das Implantat derart aufeinander abgestimmt sind, dass der Gewebekleber mit freien Aminogruppen auf der Oberfläche des Implantats reagieren und kovalente Bindungen ausbilden kann, um eine hohe Haftkraft zu erzielen. Hierzu kann das Implantat zumindest oberflächlich aus einem entsprechenden Polymer mit freien Aminogruppen bestehen. Alternativ oder zusätzlich kann das Implantat eine Beschichtung aufweisen, die freie Aminogruppen bereitstellt, beispielsweise eine Polyimin-Beschichtung.

Weitere Merkmale der Erfindung ergeben sich aus den Ansprüchen, den Figuren und der Figurenbeschreibung.
- Fig. 1: eine Bildungsreaktion von methacrylierter Gelatine (GelMA);
- Fig. 2: eine Vernetzungsreaktion von GelMA und modifizierter Hyaluronsäure (HA-NB) zur Herstellung eines ersten Netzwerks;
- Fig. 3: ein durch Quervernetzung des ersten Netzwerks hergestelltes zweites Netzwerk;
- Fig. 4: eine Kopplungsreaktion von Thapsigargin an GelMA;
- Fig. 5: eine Kopplungsreaktion eines (Meth)Acrylat-modifizierten Thapsigargin-Derivats an GelMA; und
- Fig. 6: eine Prinzipdarstellung eines erfindungsgemäßen ophthalmologischen Implantationssystems.

### Bevorzugte Ausführung der Erfindung

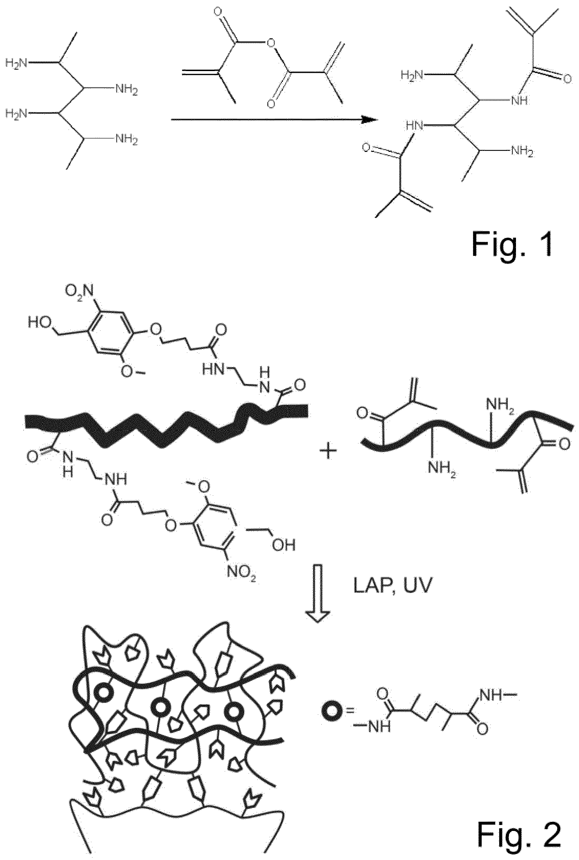

Aus Hong, Y., Zhou, F., Hua, Y. et al. (A strongly adhesive hemostatic hydrogel for the repair of arterial and heart bleeds. Nat Commun 10, 2060 (2019)) ist ein Hydrogel-Gewebekleber bekannt, der der Zusammensetzung der extrazellulären Matrix von biologischen Bindegeweben ähnelt und zur Anwendung in einem Behandlungsverfahren geeignet ist, bei welchem eine Augenlinse eines menschlichen oder tierischen Patienten durch ein ophthalmologisches Implantat ersetzt und das ophthalmologische Implantat mittels des Gewebeklebers stoffschlüssig mit einem Kapselsack des Patienten verbunden wird. Das Behandlungsverfahren kann beispielsweise eine Kataraktoperation sein. Dieser Gewebekleber bildet ein Hydrogel und besteht aus etwa 5 % methacrylisierter Gelatine (GelMA) und etwa 1,25 % N-(2-Aminoethyl)-4-(4-(hydroxymethyl)-2-methoxy-5-nitrosophenoxy)butanamid (NB), das mit dem Glykosaminoglykan Hyaluronsäure (HA) verbunden ist (HA-NB). Fig. 1 zeigt schematisch eine Bildungsreaktion von GelMA, bei welcher Gelatine mit Methacrylanhydrid vermischt und gegebenenfalls unter Rühren für 48 Stunden in DPBS (Dulbecco's phosphate-buffered saline) auf 50 °C gehalten wird. NB wird seinerseits an HA gebunden und mit GelMA vernetzt, um ein erstes Netzwerk GelMA/HA-NB auszubilden. Die Vernetzungsreaktion wird durch UV-Photoaktivierung des Polymerisationsinitiators Lithium-Phenyl-2,4,6-trimethylbenzoylphosphinat (LAP) gestartet (0,1 %). Die Vernetzungsreaktion von GelMA und modifizierter Hyaluronsäure (HA-NB) zur Herstellung eines ersten Netzwerks ist schematisch in Fig. 2 gezeigt.

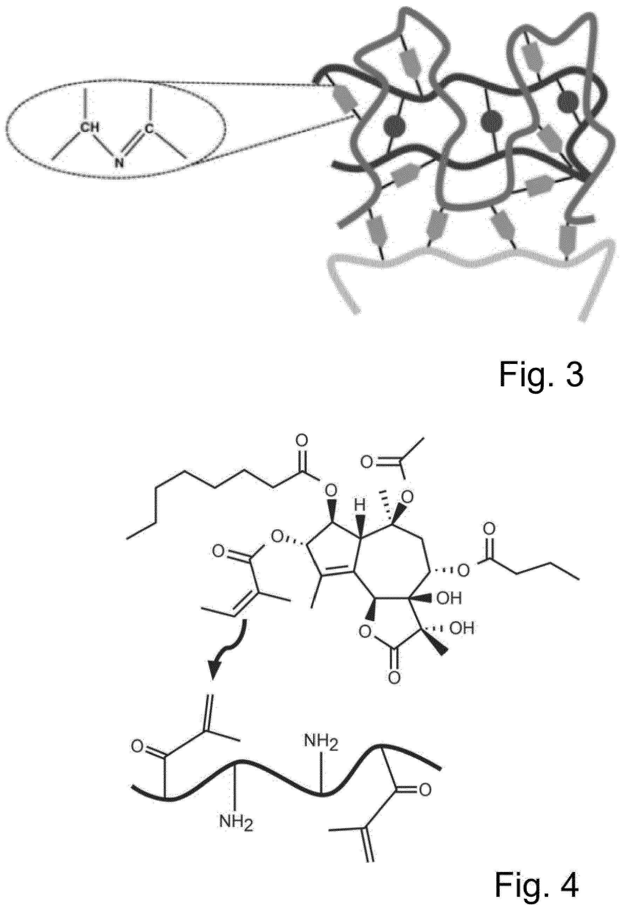

Durch die UV-Bestrahlung wandeln sich Hydroxymethyl-Gruppen von NA in Ketogruppen um, die mit freien Aminogruppen des GelMA zu Schiff'schen Basen reagieren und hierbei ein zweites Netzwerk ausbilden. Das resultierende zweite Netzwerk ist in Fig. 3 schematisch dargestellt. Der resultierende Gewebekleber bindet stark an feuchte biologische Gewebeoberflächen nach UV-Photoaktivierung der LAP.

Das in Fig. 4 gezeigte Zytotoxin Thapsigargin, ein Inhibitor des Calcium-ATPase-Inhibitors des endoplasmatischen Retikulums, reduziert das Zellwachstum im Kapselsack bei niedrigen Konzentrationen (100 nM) stark und induziert bei höheren Konzentrationen (10-100 µM) den Zelltod. Daher kann es grundsätzlich zur Vermeidung von PCO und Fibrose verwendet werden. Freies Thapsigargin, das in das Kammerwasser der Vorderkammer abgegeben wird, kann aber die Epithelzellschicht der Hornhaut schädigen.

Um eine unkontrollierte Abgabe des Thapsigargins zu verhindern, werden gemäß Fig. 4 die Acrylatgruppen des Thapsigargins kovalent an GelMA gebunden. Dazu wird das Thapsigargin dem vorstehend genannten Gewebekleber beigemischt, so dass das Thapsigargin durch die UV- und LAP-Anwendung, die zur Aushärtung des Gewebeklebers verwendet wird, ebenfalls kovalent an das GelMA bindet.

Das gebundene Thapsigargin kann keine toxische Wirkung mehr entfalten, weil es dazu in die Zellen gelangen muss. Das Kammerwasser des Auges enthält Matrix-Metalloproteinasen, deren Konzentration bei der Kataraktoperation aufgrund eines erhöhten TGFβ-Spiegels ansteigt. Matrix-Metalloproteinasen sind Gelatinasen, die Kollagene und Gelatine verdauen. In der Gegenwart dieser Matrix-Metalloproteinasen wird das thapsigarginhaltige GelMA mit der Zeit abgebaut, wodurch eine kontrollierte Freisetzung des Thapsigargins im Sinne eines Wirkstoffabgabesystems realisiert werden kann. Durch die Einbindung in den Gewebekleber wird nur in der unmittelbaren Nähe des Gewebeklebers und damit in der unmittelbaren Nähe von PCO und Fibrose verursachenden Zellen eine geringe Menge Thapsigargin über einen einstellbaren Zeitraum freigesetzt, ohne andere Gewebe zu schädigen.

Es ist zu betonen, dass auch andere pharmakologische Wirkstoffe vorgesehen sein können, die in diesen oder in andere geeignete Gewebekleber eingelagert sind, kovalent an diese gebunden sind oder anderweitig eine Zusammensetzung mit diesen bilden. Die Zusammensetzung kann vor, während und/oder nach der endgültigen Mischung der Gewebekleberkomponenten und der Aushärtung durch LAP/UV hergestellt werden. Der oder die Wirkstoffe müssen nicht unbedingt an die Acrylgruppen des Gewebeklebers binden, auch eine Bindung an die freien Aminogruppen des GelMA kann alternativ oder zusätzlich vorgesehen sein.

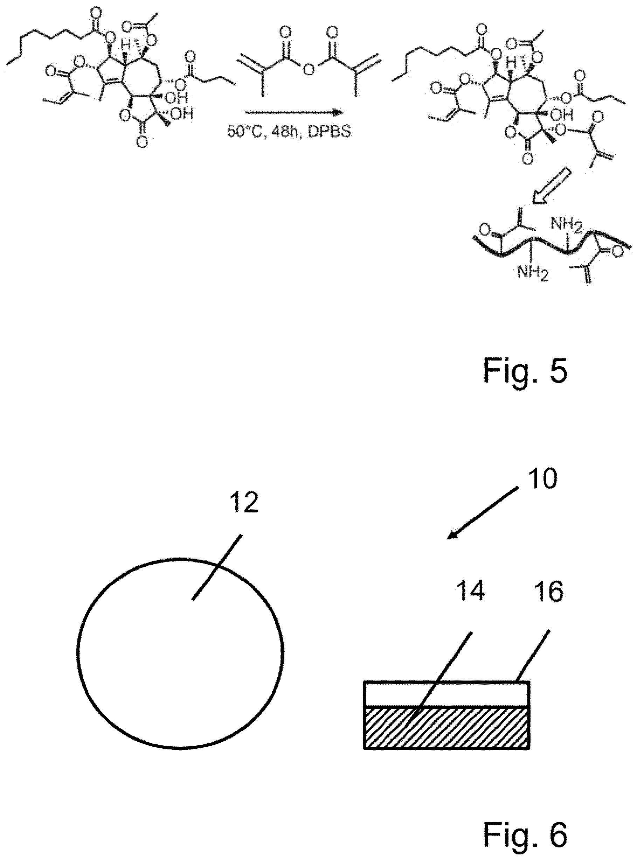

Fig. 5 zeigt eine schematische Kopplung von modifiziertem Thapsigargin an GelMA. Das Thapsigargin wird im vorliegenden Fall kovalent über eine Acrylatgruppe an GelMA bzw. an den Gewebekleber 14 gebunden. Dies hat den Vorteil einer geringeren sterischen Hinderung und einer entsprechend einfacheren Reaktionsführung mit höherem und schnellerem Umsatz. Dazu wird das Thapsigargin zunächst mit Methacrylanhydrid bei etwa 50 °C für 48 Stunden in phosphatgepufferter Salzlösung (DPBS - Dulbecco's Phosphate-Buffered Saline) derivatisiert. Die Methacrylatgruppe bindet dabei an freie OH-Gruppen des Thapsigargins. Anschließend erfolgt die kovalente Anbindung des derivatisierten Thapsigargins über diese Metharylatgruppe mit entsprechenden Metharylatgruppe der modifizierten Gelatine (GelMA). Nach der Augenoperation und der Implantation des Implantats 12 wird durch die bereits beschriebene Degradation durch Matrix-Metalloproteasen (MMP) das Thapsigargin langsam im Operationsbereich freigesetzt.

Fig. 6 zeigt eine Prinzipdarstellung eines erfindungsgemäßen ophthalmologischen Implantationssystems 10. Das Implantationssystem 10 umfasst ein ophthalmologisches Implantat 12, mittels welchem eine Augenlinse eines Patienten ersetzbar ist. Das Implantat 12 kann beispielsweise als akkommodierende Intraokularlinse ausgebildet sein. Alternativ kann das Implantat 12 aber auch ein anderer Implantattyp sein, beispielsweise eine nichtakkomodierende IOL, eine (ggf. akkommodierende) IOL mit einem oder mehreren haptischen Teilen oder ein künstlicher Kapselsack (nicht gezeigt). Ein geeigneter künstlicher Kapselsack, in welchen seinerseits eine IOL implantierbar ist, ist beispielsweise aus der US 8,900,300 B1 bekannt. Weiterhin umfasst das Implantationssystem 10 einen Gewebekleber 14, mittels welchem das ophthalmologische Implantat 12 nach seiner Implantation stoffschlüssig mit einem Kapselsack des Patienten verbindbar ist. Der Gewebekleber 14 kann wie vorstehend beschrieben ausgebildet sein und bis zu seiner Verwendung in einer geeigneten Verpackung 16 gelagert werden. Alternativ kann der Gewebekleber 14 bereits auf dem Implantat 12 aufgebracht sein. In diesem Fall werden das Implantat 12 und der Gewebekleber 14 vorzugsweise derart gelagert, dass ein verfrühtes Aushärten des Gewebeklebers 14, das heißt ein Aushärten während der Lagerung bzw. vor der Implantation, verhindert wird.

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, Einwaagefehlern und dergleichen - als vom Rahmen der Erfindung mitumfasst anzusehen.

### Bezugszeichenliste

- 10: Implantationssystem
- 12: Implantat
- 14: Gewebekleber
- 16: Verpackung

## Patentansprüche

1. Gewebekleber (14) zur Anwendung in einem Behandlungsverfahren, bei welchem einem menschlichen oder tierischen Patienten ein ophthalmologisches Implantat (12) implantiert und das ophthalmologische Implantat (12) mittels des Gewebeklebers (14) zur Verringerung des Risikos von posteriorer Kapselopazifikation (PCO) und Fibrose zumindest teilweise stoffschlüssig mit Augengewebe des Patienten verbunden wird, wobei der Gewebekleber (14) im nicht-ausgehärteten Zustand als interpenetrierendes Netzwerk und/oder als semi-interpenetrierendes Netzwerk vorliegt,
**dadurch gekennzeichnet, dass**
dieser als Zusammensetzung mit einem Wirkstoffabgabesystem bereitgestellt ist, welches dazu ausgebildet ist, im implantierten Zustand des Gewebeklebers (14) wenigstens einen pharmakologischen Wirkstoff abzugeben, wobei der wenigstens eine Wirkstoff zum Fördern und/oder Hemmen wenigstens eines Aspekts aus einer Gruppe, die Proliferation, Migration und Differenzierung von im menschlichen oder tierischen Auge vorkommenden Zellen aufweist, ausgebildet ist und wobei
der wenigstens eine Wirkstoff ausgewählt ist aus einer Gruppe, die 5-Fluoruracil, Thapsigargin, Paclitaxel, Wachstumsfaktoren, insbesondere TGFβ, und Angiogenesehemmer sowie (Meth)Acrylat-modifizierte Derivate, Isomere und beliebige Mischungen hiervon umfasst.

2. Gewebekleber (14) nach Anspruch 1,
**dadurch gekennzeichnet, dass**
dieser als Zusammensetzung mit einem Wirkstoffabgabesystem bereitgestellt ist, welches dazu ausgebildet ist, im implantierten Zustand des Gewebeklebers (14) wenigstens einen pharmakologischen Wirkstoff kontrolliert abzugeben und/oder dass der Gewebekleber (14) als Zusammensetzung mit einem am Gewebekleber (14) immobilisierten pharmakologischen Wirkstoff bereitgestellt ist.

3. Gewebekleber (14) nach Anspruch 1 oder 2,
**dadurch gekennzeichnet, dass**
der Wirkstoff kovalent, insbesondere über eine (Meth)Acrylat-Gruppe, an den Gewebekleber (14) gebunden ist und/oder dass der Wirkstoff und der Gewebekleber (14) als interpenetrierendes Netzwerk vorliegen.

4. Gewebekleber (14) nach einem der Ansprüche 1 bis 3,
**dadurch gekennzeichnet, dass**
dieser im nicht-ausgehärteten Zustand als Hydrogel vorliegt und/oder durch wenigstens einen Mechanismus aus der Gruppe anaerob aushärtend, durch UV-Licht aushärtend, anionisch aushärtend, mit Aktivator aushärtend, durch Feuchtigkeit aushärtend und warmhärtend auszuhärten ist.

5. Gewebekleber (14) nach einem der Ansprüche 1 bis 4,
**dadurch gekennzeichnet, dass**
dieser ein MeTro (methacryliertes rekombinantes Tropoelastin) Präpolymer und/oder ein GelMA (methacrylierte Gelatine)/NB (N-(2-aminoethyl)-4-(4-(hydroxymethyl)-2-methoxy-5-nitrosophenoxy)butanamid)-enthaltendes Polymer umfasst, welches vorzugsweise mit einem Biopolymer, insbesondere Hyaluronsäure (HA) verbunden ist.

6. Ophthalmologisches Implantationssystem (10), umfassend ein ophthalmologisches Implantat (12) zur Implantation in einem menschlichen oder tierischen Auge und einen Gewebekleber (14), mittels welchem das ophthalmologische Implantat (12) zur Verringerung des Risikos von posteriorer Kapselopazifikation (PCO) und Fibrose zumindest teilweise stoffschlüssig mit Augengewebe des Patienten verbindbar ist,
**dadurch gekennzeichnet, dass**
der Gewebekleber (14) gemäß einem der Ansprüche 1 bis 5 ausgebildet ist.

7. Ophthalmologisches Implantationssystem (10) nach Anspruch 6,
**dadurch gekennzeichnet, dass**
das ophthalmologische Implantat (12) eine Intraokularlinse oder ein künstlicher Kapselsack ist.

8. Ophthalmologisches Implantationssystem (10) nach Anspruch 6 oder 7,
**dadurch gekennzeichnet, dass**
das ophthalmologische Implantat (12) an seiner Außenseite freie Aminogruppen umfasst, über welche das ophthalmologische Implantat (12) vermittels des Gewebeklebers (14) stoffschlüssig mit dem Augengewebe verbindbar ist.

## Claims

1. Tissue adhesive (14) for use in a treatment method in which an ophthalmological implant (12) is implanted in a human or animal patient and the ophthalmological implant (12) is at least partly cohesively bonded to the tissue of the patient's eye by means of the tissue adhesive (14) in order to reduce the risk of posterior capsular opacification (PCO) and fibrosis, wherein the tissue adhesive (14) in the uncured state takes the form of an interpenetrating network and/or of a semi-interpenetrating network,
**characterized in that**
it is provided as a composition with an active ingredient release system which is designed to release at least one active pharmacological ingredient in the implanted state of the tissue adhesive (14), wherein
the at least one active ingredient is designed to promote and/or inhibit at least one aspect from a group including proliferation, migration and differentiation of cells that occur in the human or animal eye, and wherein
the at least one active ingredient is selected from a group comprising 5-fluorouracil, thapsigargin, paclitaxel, growth factors, especially TGFβ, and angiogenesis inhibitors, and also (meth)acrylate-modified derivatives, isomers and any mixtures thereof.

2. Tissue adhesive (14) according to Claim 1,
**characterized in that**
it is provided as a composition with an active ingredient release system designed, in the implanted state of the tissue adhesive (14), to release at least one active pharmacological ingredient in a controlled manner, and/or **in that** the tissue adhesive (14) is provided as a composition with an active pharmacological ingredient immobilized on the tissue adhesive (14).

3. Tissue adhesive (14) according to Claim 1 or 2,
**characterized in that**
the active ingredient is bonded covalently to the tissue adhesive (14), especially via a (meth)acrylate group, and/or **in that** the active ingredient and the tissue adhesive (14) take the form of an interpenetrating network.

4. Tissue adhesive (14) according to any of Claims 1 to 3,
**characterized in that**
in the uncured state it takes the form of a hydrogel and/or can be cured by at least one mechanism from the group of anaerobically curing, UV light-curing, anionically curing, activator-curing, moisture-curing and thermally curing.

5. Tissue adhesive (14) according to any of Claims 1 to 4,
**characterized in that**
it comprises a MeTro (methacrylated recombinant tropoelastin) prepolymer and/or a GelMA (methacrylated gelatin)/NB (N-(2-aminoethyl)-4-(4-(hydroxymethyl)-2-methoxy-5-nitrosophenoxy)butanamide)-containing polymer, preferably combined with a biopolymer, especially hyaluronic acid (HA).

6. Ophthalmological implantation system (10) comprising an ophthalmological implant (12) for implantation in a human or animal eye and a tissue adhesive (14) by means of which the ophthalmological implant (12) is at least partly cohesively bondable to the patient's eye tissue in order to reduce the risk of posterior capsular opacification (PCO) and fibrosis,
**characterized in that**
the tissue adhesive (14) is designed according to any of Claims 1 to 5.

7. Ophthalmological implantation system (10) according to Claim 6,
**characterized in that**
the ophthalmological implant (12) is an intraocular lens or an artificial capsular bag.

8. Ophthalmological implantation system (10) according to Claim 6 or 7,
**characterized in that**
the ophthalmological implant (12) comprises, on the outside thereof, free amino groups via which the ophthalmological implant (12) is cohesively bondable to the eye tissue by means of the tissue adhesive (14).

## Revendications

1. Adhésif pour tissu (14) destiné à être utilisé dans une méthode de traitement dans laquelle un implant ophtalmologique (12) est implanté chez un patient humain ou animal et l'implant ophtalmologique (12) est relié au moins partiellement par liaison de matière au moyen de l'adhésif pour tissu (14) au tissu oculaire du patient pour la réduction du risque d'une opacification capsulaire postérieure (PCO) et d'une fibrose, l'adhésif pour tissu (14) se trouvant dans l'état non durci sous forme d'un réseau interpénétrant et/ou d'un réseau semi-interpénétrant,
**caractérisé en ce que**
celui-ci est mis à disposition sous forme de composition présentant un système de libération de principe actif qui est conçu pour libérer, dans l'état implanté de l'adhésif pour tissu (14), au moins un principe actif pharmacologique,
ledit au moins un principe actif étant conçu pour favoriser et/ou inhiber au moins un aspect d'un groupe présentant la prolifération, la migration et la différenciation de cellules se trouvant dans l'œil humain ou animal et
ledit au moins un principe actif étant choisi dans un groupe comprenant le 5-fluorouracile, la thapsigargine, le paclitaxel, les facteurs de croissance, en particulier le TGFβ, et les inhibiteurs de l'angiogenèse ainsi que des dérivés modifiés par (méth)acrylate, des isomères et des mélanges quelconques de ceux-ci.

2. Adhésif pour tissu (14) selon la revendication 1,
**caractérisé en ce que**
celui-ci est mis à disposition sous forme de composition présentant un système de libération de principe actif, qui est conçu pour libérer de manière prolongée, dans l'état implanté de l'adhésif pour tissu (14), au moins un principe actif pharmacologique et/ou **en ce que** l'adhésif pour tissu (14) est mis à disposition sous forme de composition présentant un principe actif pharmacologique immobilisé sur l'adhésif pour tissu (14).

3. Adhésif pour tissu (14) selon la revendication 1 ou 2,
**caractérisé en ce que**
le principe actif est lié à l'adhésif pour tissu (14) par covalence, en particulier par l'intermédiaire d'un groupe (méth)acrylate et/ou **en ce que** le principe actif et l'adhésif pour tissu (14) se trouvent sous forme de réseau interpénétrant.

4. Adhésif pour tissu (14) selon l'une des revendications 1 à 3,
**caractérisé en ce que**
celui-ci se trouve dans l'état non durci sous forme d'hydrogel et/ou est durcissable par au moins un mécanisme du groupe constitué par un durcissement anaérobie, un durcissement par la lumière UV, un durcissement par voie anionique, un durcissement par un activateur, un durcissement par l'humidité et un durcissement par la chaleur.

5. Adhésif pour tissu (14) selon l'une des revendications 1 à 4,
**caractérisé en ce que**
celui-ci comprend un prépolymère MeTro (tropoélastine méthacrylée recombinante) et/ou un polymère contenant du GelMA (gélatine méthacrylée)/NB (N-(2-aminoéthyl)-4-(4-(hydroxyméthyl)-2-méthoxy-5-nitrosophénoxy)butanamide) qui est de préférence lié à un biopolymère, en particulier l'acide hyaluronique (HA).

6. Système d'implantation ophtalmologique (10), comprenant un implant ophtalmologique (12) destiné à être implanté dans un œil humain ou animal et un adhésif pour tissu (14), au moyen duquel l'implant ophtalmologique (12) peut être lié au moins partiellement par liaison de matière au tissu oculaire du patient pour la réduction du risque d'une opacification capsulaire postérieure (PCO) et d'une fibrose,
**caractérisé en ce que**
l'adhésif pour tissu (14) est conçu selon l'une des revendications 1 à 5.

7. Système d'implantation ophtalmologique (10) selon la revendication 6,
**caractérisé en ce que**
l'implant ophtalmologique (12) est une lentille intraoculaire ou un sac capsulaire artificiel.

8. Système d'implantation ophtalmologique (10) selon la revendication 6 ou 7,
**caractérisé en ce que**
l'implant ophtalmologique (12) comprend sur sa face externe des groupes amino libres, par l'intermédiaire desquels l'implant ophtalmologique (12) peut être relié par liaison de matière au tissu oculaire au moyen de l'adhésif pour tissu (14).
